# EUROPÄISCHE PATENTANMELDUNG

(11) **EP 0 931 791 A1**
(43) Veröffentlichungstag der Anmeldung: **28.07.1999**
(21) Anmeldenummer: 99101041.4
(22) Anmeldetag: 21.01.1999
(51) Int. Cl.: C07D 233/78, C08G 18/78, C08G 18/62

(54) **Hydantoingruppenhaltige Diisocyanate und diese beinhaltende Polyurethane**

(30) Priorität: 23.01.1998 DE 19802547
(71) Anmelder: BASF AKTIENGESELLSCHAFT, 67056 Ludwigshafen (DE)
(72) Erfinder: Graf, Hermann, Dr., 67112 Mutterstadt (DE); Rotermund, Udo, Dr., 01990 Ortrand (DE); Mohrhardt, Günter, 67346 Speyer (DE)
(74) Vertreter: Isenbruck, Günter, Dr.

(57) **Zusammenfassung**

Diisocyanat der Formel (I) 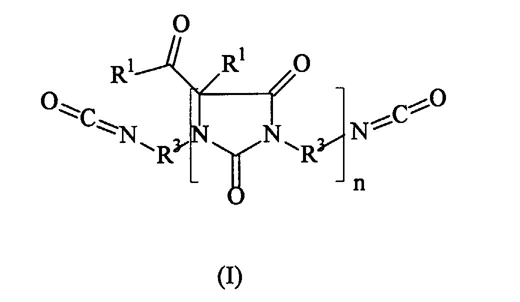 wobei R¹ ein C₁- bis C₁₀-Kohlenwasserstoffrest und R³ eine C₁- bis C₁₂-Kohlenwasserstoffgruppe bedeutet und n einer ganzen Zahl im Bereich von 1 bis 10 entspricht.

## Beschreibung

Die Erfindung betrifft hydantoingruppenhaltige Diisocyanate, ein Verfahren zu deren Herstellung, hydantoingruppenhaltige Diisocyanate beinhaltende Polyurethane sowie die Verwendung dieses Polyurethanes zur Herstellung von Fasern, Folien, Schäumen, Formmassen, Beschichtungen und Lacken als auch Fasern, Folien, Schäumen, Formmassen, Beschichtungen und Lacke, die dieses Polyurethan enthalten.

Polyurethane entstehen durch die Umsetzung von Polyisocyanaten mit Polyolen. Aufgrund der vielfältigen Umsetzungsmöglichkeiten der Isocyanatgruppe und ihrer hohen Reaktivität werden die Polyisocyanate für die Herstellung von Schaumstoffen, Fasern, Folien, Lacken und Anstrichfarben verwendet. Beispielsweise können Polyurethan Hartschaumstoffe zum Ausschäumen von Kältemöbeln oder Heizelementen verwendet werden, da sie sich durch eine niedrige Wärmeleitfähigkeit auszeichnen. Durch die Wahl der Rohstoffe lassen sich die Eigenschaften der Hartschaumstoffe in weiten Bereichen variieren.

Eine Zusammenfassende Übersicht über Polyurethane, deren Herstellungsweise und Verwendung ist in Becker/Braun, Kunststoff Handbuch, Band 7, Polyurethane, 3. Aufl. 1993, Carl Hanser Verlag beschrieben.

EP-A 0 744 419, 0 744 424, 0 744 425 sowie 0 744 427 offenbaren ein Verfahren zur Synethese von hydantoinhaltigen Polyurethanprepolymeren. Diesen Verfahren ist gemein, daß die Hydantoingruppen im Prepolymeren durch eine bei höheren Temperaturen erfolgende Ringschlußkondensationsreaktion entstehen. Weiterhin entstehen gemäß dieses Verfahrens die Hydantoingruppen nur an Prepolymeren, deren Isocyanatgruppen durch eine Schutzgruppe blockiert sind.

Die in diesen Druckschriften offenbarte Schutzgruppentechnik ist nachteilhaft, da mit ihr auf dem Wege zur fertigen Polyurethan zusätzliche Syntheseschritte notwendig sind.

Es ist daher erfindungsgemäß Aufgabe, ein Verfahren zur Synthese von hydantoingruppenhaltigen Prepolymeren und Polyurethanen sowie dementsprechende Prepolymere und Polyurethane zur Verfügung zu stellen, wobei auch die Syntheseschritte zur Einführung und Entfernung entsprechender Schutzgruppen verzichtet werden kann.

Weiterhin ist es eine erfindungsgemäße Aufgabe, ein Verfahren zur Synthese von hydantoingruppenhaltigen Prepolymeren und Polyurethanen sowie dem entsprechenden Prepolymere und Polyurethane zur Verfügung zu stellen, in dem das hydantoingruppenhaltige Prepolymer oder Polyurethan gebildet wird und sich gleichzeitig ein Flammschutzmittel bildet.

Die Aufgabe wird erfindungsgemäß gelöst durch ein Diisocyanat der Formel (I) 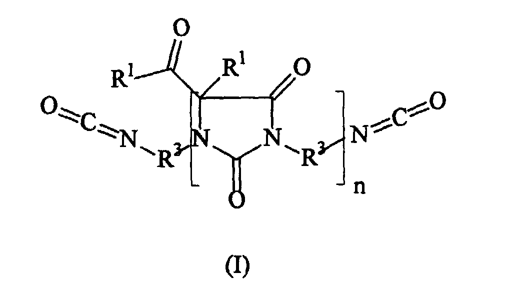 wobei R¹ ein C₁- bis C₁₀-Kohlenwasserstoffrest und R³ eine C₁- bis C₁₂-Kohlenwasserstoffgruppe und n einer ganzen Zahl im Bereich von 1 bis 10 entspricht.

R¹ ist bevorzugt ein C₁- bis C₆-Alkylrest insbesondere ein Methyl, Ethyl, Propyl, oder Butylrest, oder ein Arylrest, besonders bevorzugt ein Phenylrest. R³ ist bevorzugt eine Alkylengruppe mit 3 bis 12 Kohlenstoffatomen, eine Cycloalkenylgruppe mit 3 bis 12 Kohlenstoffatomen oder eine Arylengruppe mit 7 bis 12 Kohlenstoffatomen, insbesondere eine unverzweigte Alkylgruppe mit 4 bis 8 Kohlenstoffatomen, besonders bevorzugt mit 6 Kohlenstoffatomen, die sich endständig an der Isocyanatgruppe befinden.

Weiter wird die Aufgabe gelöst durch ein Verfahren zur Herstellung von Diisocyanat, insbesondere der Formel (I), bei dem Phospholene der Formel (II), in der R¹ wie vorstehend beschrieben und R² eine C₁- bis C₁₀-Kohlenwasserstoffgruppe bedeutet, mit Diisocyanaten der Formel (III), mit R³ wie zuvor definiert, umgesetzt werden, wobei ein Phosphat der Formel (IV) entsteht.

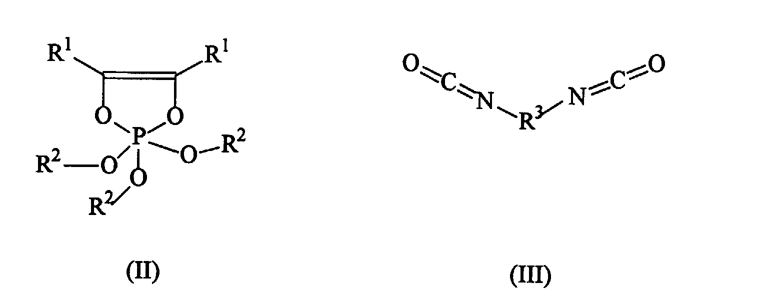

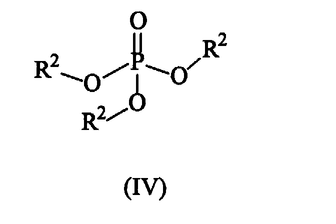

R¹ und R² können dabei gleich oder voneinander verschieden sein.

Darüber hinaus wurde überraschender Weise festgestellt, daß sowohl die hydantoinhaltigen Diisocyanate der Formel (I) als auch die daraus erhaltenen Polyurethane gegenüber dem Stand der Technik aufgeführten hydantoinhaltigen Diisocyanate und Polyurethane wesentlich temperaturbeständiger sind.

Als organische Diisocyanate der allgemeinen Formel (III) können alle bekannten geeigneten, aliphatischen, cycloaliphatischen und aromatischen Verbindungen mit mehr als einer Isocyanatgruppe verwendet werden. Bevorzugte organische Diisocyanate sind geradkettige oder verzweigte Alkylendiisocyanate mit 1 bis 12 Kohlenstoffatomen, wie 1,12-Dodecandiisocyanat, 2-Ethyl-1,4-butylendiisocyanat, 2-Methyl-1,5-pentylendiisocyanat, 1,4-Butylendiisocyanat, 1,6-Hexamethylen-diisocyanat (HDI); cycloaliphatische Diisocyanate mit 3 bis 12 Kohlenstoffatomen, wie 1,3-Cyclohexyldiisocyanat, 1,4-Cyclohexyldiisocyanat, 1-Isocyanato-3,3,5-trimethyl-5-isocyanatomethylcyclo-hexan (IPDI), 2,4- und 2,6-Hexahydrotoluylendiisocyanat und die entsprechenden Isomerengemische, 4,4'-, 2,2'- und 2,4'-Dicyclohexylmethyldiisocyanat sowie die entsprechenden Isomerengemische und aromatische Diisocyanate mit 7 bis 12 Kohlenstoffatomen wie 2,4- und 2,6-Toluyldiisocyanat (TDI) und deren Isomerengemische, 4,4'-, 2,2'- und 2,4'-Diphenylmethandiisocyanat (MDI), sowie deren Isomerengemische, Mischungen aus 4,4'-, 2,2'- Diphenylmethandiisocyanaten, Polyphenylpolymethylenpolyisocyanate (Polymer MDI), Mischungen aus 4,4'-, 2,2'- und 2,4'-Diphenylmethandiisocyanaten und Polyphenylpolymethylenpolyisocyanaten (Roh-MDI) und Mischungen aus Roh-MDI und Toluylendiisocyanaten. Besonders bevorzugt werden 1,6-Hexamethylendiisocyanat (HDI), 2,4- und 2,6-Toluyldiiso-cyanat (TDI) und deren Isomerengemische, Mischungen aus 4,4'-, 2,2'- Diphenylmethandiisocyanaten (MDI) und Polyphenylpolymethylen-polyisocyanate (Polymer MDI) verwendet.

In einer weiteren Ausführungsform des erfindungsgemäßen Verfahrens wird das bei der Reaktion entstehende Phosphat bei der Formel (IV) zumindest teilweise, bevorzugt vollständig aus dem Reaktionsgemisch entfernt.

Weiterhin werden in dem erfindungsgemäßen Verfahren die Phospholene der Formel (II) mit den Diisocyanaten der Formel (III) umgesetzt, wobei die Diisocyanate der Formel (III) vorzugsweise in einem Überschuß, besonders bevorzugt mindestens in einem zweifachen Überschuß eingesetzt werden.

Aus dieser Umsetzung entsteht eine Zusammensetzung, die mindestens ein Diisocyanate der Formel (I), ein Diisocyanat der Formel (III) und gegebenfalls ein Phosphat der Formel (IV) aufweist.

Der Anteil des als Flammschutzmittel wirkenden Phosphats der Formel (IV) an der Zusammensetzung richtet sich danach, in wie weit das Phosphat der Formel (IV) aus der Zusammensetzung, wie zuvor beschrieben, entfernt wurde, wobei das Phosphat der Formel (IV) seine Flammschutzwirkung mit steigenden Anteil an der Zusammensetzung stärker entfaltet.

Der Anteil des als Flammschutzmittel wirkenden Phosphats der Formel (IV) an der gesamten Zusammensetzung wird so gewählt, daß in dem als Endprodukt entstehenden Polyurethan so viel Phosphat der Formel (IV) enthalten sind, daß ein ausreichender Flammschutz gewährleistet ist.

Der Anteil des Phosphats der Formel (I) beträgt 0,1 bis 25 und bevorzugt 0,5 bis 7 Gew.-%, bezogen auf das gesamte Polyurethan und besonders bevorzugt bezogen auf die Polyurethan-A Komponente.

Ferner kann durch den Anteil des Phosphats der Formel (IV) die Viskosität der Zusammensetzung gesteuert werden.

Die Anteile von (a) zu (b) und zu dem Diisocyanate der Formel (I) betragen in einer bevorzugten Ausführungsform der Erfindung 1:1:1 bis 6:1:1 und vorzugsweise 1:1:1 bis 4:1:1.

Erfindungsgemäß ist es besonders bevorzugt mittels des Phosphats der Formel (IV) die Viskosität des noch nicht ausgehärteten Polyurethans bzw. der Polyurethankomponenten zu variieren. Dieses ist insbesondere vorteilhaft, wenn das Polyurethan zum ausfüllen, vorzugsweise zum ausschäumen von Formen mit komplizierten Aufbau verwendet wird. Die Steuerung der Viskosität in Richtung leicht fließender Flüssigkeiten erfolgt dadurch, in dem der Anteil des Phosphats der Formel (IV) an dem noch nicht polymerisierten Polyurethan oder an den Polyurethankomponenten erhöht wird.

In einer bevorzugten Ausführungsform beträgt der Anteil des Phosphats der Formel (IV) an allen eingesetzten Komponenten außer Komponente (a) 0,5 bis 30 und bevorzugt 3 bis 20 Gew.-%.

Die Viskosität der zur Polyurethansynthese eingesetzten Komponenten außer der Komponente (a) beträgt 200 bis 10000, bevorzugt 600 bis 3000 mPa · s/20° C.

Weiterhin ist erfindungsgemäß ein Polyurethan erhältlich aus mindestens den Bestandteilen: mindestens ein Diisocyanat der Formel (I) oder eine Zusammensetzung mindestens enthaltend Diisocyanat der Formel (I), Diisocyanat der Formel (III) oder deren Mischung als organisches Polyisocyanat (a) und mindestens ein Diol als organische Verbindung mit mindestens zwei reaktiven Wasserstoffatomen (b).

Vorzugsweise handelt es sich bei dem Bestandteil (b) mindestens um ein Acrylatharz mit mindestens zwei Hydroxylgruppen pro Molekül und einem Molekulargewicht von als Gewichtsmittel (M_{w}) von 300 bis 20.000, bevorzugt 10.000 bis 15.000 und besonders bevorzugt 2.000 bis 10.000 g/mol.

In dem erfindungsgemäßen Polyurethans ist der Bestandteil (b) vorzugsweise ein Acrylatharz, vorzugsweise wenn das erfindungsgemäße Polyurethan als gut härtbarer Lack verwendet wird.

Darüber ist es bevorzugt, das in dem Verfahren zur Herstellung des erfindungsgemäßen Polyurethans und somit auch in dem daraus erhältlichen Polyurethan neben den Bestandteilen (a) und (b) Kettenverlängerungs- und/oder Vernetzungsmittel (c) oder Katalysatoren (d) oder Treibmittel (e) oder Flammschutzmittel (f), Hilfsmittel- und/oder Zusatzstoffe (g) oder eine mindestens zwei dieser Bestandteile enthaltende Mischung eingesetzt werden bzw. vorliegen.

In einer anderen Ausführungsform des erfindungsgemäßen Verfahrens wird das erfindungsgemäße Polyurethan durch die Umsetzung von organischem Polyisocyanaten (a) mit organischen Verbindungen mit mindestens zwei reaktiven Wasserstoffatomen (b) und Kettenverlängerungs- und Vernetzungsmitteln (c) hergestellt.

In einer weiterhin erfindungsgemäß bevorzugten Ausführungsform wird das erfindungsgemäße Polyurethan durch die Umsetzung von organischen Polyisocyanaten (a) mit organischen Verbindungen mit mindestens zwei reaktiven Wasserstoffatomen (b) und mit weiteren Katalysatoren (d) hergestellt.

In einer anderen Ausführungsform des erfindungsgemäßen Verfahrens zur Herstellung des erfindungsgemäßen Polyurethans werden organische Polyisocyanate (a) mit organischen Verbindungen mit mindestens zwei reaktiven Wasserstoffatomen (b) und Kettenverlängerungs- und Vernetzungsmitteln (c) und mit Katalysatoren (d) umgesetzt.

Eine weitere Ausführungsform des erfindungsgemäßen Verfahrens zur Herstellung von Polyurethan zeichnet sich dadurch aus, daß die organischen Polyisocyanate (a) mit den organischen Verbindungen mit mindestens zwei reaktiven Wasserstoffatomen (b) und Treibmittel (e) umgesetzt werden.

In einer weiteren Ausführungsform des erfindungsgemäßen Verfahrens werden die organischen Polyisocyanate (a) mit den organischen Verbindungen mit mindestens zwei reaktiven Wasserstoffatomen (b) und mit Kettenverlängerungs- und Vernetzungsmitteln (c) sowie mit Katalysatoren (d) zur Herstellung der erfindungsgemäßen Polyurethane umgesetzt.

In einer weiteren Ausführungsform des erfindungsgemäßen Verfahrens werden die erfindungsgemäßen Polyurethane durch die Umsetzung von organischen Polyisocyanaten (a) mit organischen Verbindungen mit mindestens zwei reaktiven Wasserstoffatomen (b) sowie mit Flammschutzmitteln (f) hergestellt.

In einer anderen Ausführungsform des erfindungsgemäßen Verfahrens werden die erfindungsgemäßen Polyurethane durch Umsetzung von organischen Polyisocyanaten (a) mit organischen Verbindungen mit mindestens zwei reaktiven Wasserstoffatomen (b), mit Kettenverlängerungs- und Vernetzungsmitteln (c) und Flammschutzmitteln (f) hergestellt.

Weiterhin liegt eine Ausführungsform des erfindungsgemäßen Verfahrens dann vor, wenn durch Umsetzung von organischen Polyisocyanaten (a) mit organischen Verbindungen mit mindestens zwei reaktiven Wasserstoffatomen (b) und Hilfsmitteln und/oder Zusatzstoffen die erfindungsgemäßen Polyurethane hergestellt werden.

Darüber hinaus liegt eine Ausführungsform des erfindungsgemäßen Verfahrens vor, wenn das erfindungsgemäße Polyurethan durch die Umsetzung von organischen Polyisocyanaten (a) mit Verbindungen mit mindestens zwei reaktiven Wasserstoffatomen (b) und mit Kettenverlängerungs- und Vernetzungsmitteln (c) sowie mit Hilfsmitteln und/oder Zusatzstoffen (g) hergestellt werden.

In einer anderen Ausführungsform des erfindungsgemäßen Verfahrens wird das erfindungsgemäße Polyurethan durch die Umsetzung von organischen Polyisocyanaten (a) mit organischen Verbindungen mit mindestens zwei reaktiven Wasserstoffatomen (b) in Gegenwart von Katalysatoren (d) und Treibmitteln (e) hergestellt.

In einer anderen Ausführungsform des erfindungsgemäßen Verfahrens wird das erfindungsgemäße Polyurethan durch die Umsetzung von organischen Polyisocyanaten (a) mit organischen Verbindungen mit mindestens zwei reaktiven Wasserstoffatomen (b) und mit Kettenverlängerungs- und Vernetzungsmitteln (c) in Gegenwart von Katalysatoren (d) und Treibmitteln (e) hergestellt.

In einer weiteren Ausführungsform des erfindungsgemäßen Verfahrens wird das erfindungsgemäße Polyurethan durch die Umsetzung von organischen Polyisocyanaten (a) und Verbindungen mit mindestens zwei reaktiven Wasserstoffatomen (b) und mit Kettenverlängerungs- und Vernetzungsmitteln (c) in Gegenwart von Flammschutzmitteln (f) und Treibmitteln (e) hergestellt.

In einer anderen Ausführungsform des erfindungsgemäßen Verfahrens werden die erfindungsgemäßen Polyurethane durch Umsetzung von organischen Polyisocyanaten (a) mit organischen Verbindungen mit mindestens zwei reaktiven Wasserstoffatomen (b) und gegebenenfalls Kettenverlängerungs- und Vernetzungsmitteln (c) in Gegenwart von Katalysatoren (d), Treibmitteln (e), Flammschutzmitteln (f) sowie gegebenenfalls Hilfsmitteln und/oder Zusatzstoffen (g) hergestellt.

Alle vorgenannten Ausführungsformen des erfindungsgemäßen Verfahrens zur Herstellung der erfindungsgemäßen Polyurethane haben die Verwendung mindestens eines Diisocyanats der Formel (I) gemein. Weiterhin gelten die Komponentenkombinationen der vorstehenden Ausführungsformen gleichfalls für die aus diesen Verfahren erhältlichen Polyurethane und -zusammensetzungen.

Die Herstellung der Phospholenvorprodukte ist in Ramirez et al., Tetrahedron 24, 1941, (1968) beschrieben.

In einer erfindungsgemäß bevorzugten Ausführungsform werden die Phospholene durch Umsetzung eines Diketons mit den zuvor definierten Resten R¹, wobei die beiden Ketogruppen vorzugsweise direkt untereinander gebunden sind, vorzugsweise Diacetyl und Benzil, mit einem Phosphit, dessen Sauerstoffe die zuvor beschrieben Reste R² tragen, vorzugsweise Trimethylphosphit, Tripropylphosphit, Triphenylphosphit und besonders bevorzugt Triethylphosphit, erhalten. Die Umsetzung erfolgt vorzugsweise in einem Temperaturbereich von -20 bis 20, bevorzugt -10 bis 10 und besonders bevozugt 0 bis 5°C. Weiterhin ist es bevorzugt, die Umsetzung in einer Inertgasatmosphäre, bevorzugt in einer Stickstoff- oder Argonatmosphäre, oder einer Mischung beider und besonders bevorzugt in einer Stickstoffatmosphäre durchzuführen. Weiterhin ist es erfindungsgemäß bei der Phospholensynthese bevorzugt, nach Ende der Umsetzung das Produkt für mehrere Stunden, vorzugsweise 2 bis 30, bevorzugt 10 bis 20 und besonders bevorzugt 12 bis 13 Stunden bei Raumtemperatur zu halten. Es hat sich als vorteilhaft erwiesen, das so erhaltene Reaktionsprodukt destillativ aufzuarbeiten. Obgleich die Phospholensynthese in jedem geeigneten Lösungsmittel durchgeführt werden kann, hat es sich als besonders vorteilhaft erwiesen, die Umsetzung ohne zusätzliches Lösungsmittel durchzuführen.

Die hydantoinhaltigen Diisocyanate der Formel (I) werden erhalten, in dem ein entsprechendes Phospholen in der Formel (II) mit einem Diisocyanat der Formel (III) umgesetzt wird. Vorzugsweise erfolgt die Umsetzung in einem Temperaturbereich von 0 bis 80, bevorzugt 20 bis 70 und besonders bevorzugt 30 bis 50°C. Weiterhin ist es in dem erfindungsgemäßen Verfahren zur Herstellung der hydantoinhaltiger Diisocyanate der Formel (I) vorteilhaft, die Reaktion in einem Lösungsmittel, vorzugsweise in einem aprotischen Lösunsmittel durchzuführen, wobei das Lösungsmittel vorzugsweise so ausgewählt werden sollte, daß es in den zuvor genannten Temperaturbereichen siedet. Besonders bevorzugt sind aprotische polare und halogenierte Lösungsmittel, wobei die halogenierten Lösungsmittel besonders bevorzugt sind. Als halogenierte Lösungsmittel kommen beispielsweise Tetrachlorkohlenstoff, Trichlormethan, Dichlormethan, Monochlormethan, 1,2-Dichlorethan, Trichlorethylen, 1,1,1-Trichlorethan in Betracht, wobei Dichlormethan besonders bevorzugt ist. Es ist besonders bevorzugt, die Umsetzung in Dichlormethan bei einer Temperatur durchzuführen, bei dem das Dichlormethan siedet. Weiterhin ist es bevorzugt, die erfindungsgemäße Umsetzung in einer Inertgasatmosphäre, vorzugsweise einer Atmosphäre aus Argon oder Stickstoff oder deren Mischung und besonders bevorzugt aus Stickstoff durchzuführen. Ferner ist es bevorzugt, sowohl das Phospholen der Formel (II) als auch das Diisocyanat der Formel (III) in Lösungsmittel aufzunehmen. Weiterhin ist es bevorzugt, daß das Diisocyanat der Formel (III) in einem geeigneten Lösungsmittel vorgelegt wird und das Phospholen der Formel (I) - gleichfalls in einem Lösungsmittel aufgenommen - über einen Zeitraum zu der vorgelegten Lösung des Diisocyanats der Formel (III) zugegeben wird. Darüber hinaus ist es bei der Synthese der erfindungsgemäßen hydantoinhaltigen Diisocyanate der Formel (I) vorteilhaft, die Reaktion durch Erhitzen innerhalb des zuvor genannten Temperaturbereiches bis zur vollständigen Umsetzung der im Unterschuß zugegebenen Komponente zu führen. Nach Beendigung der Reaktion wird das oder die Lösungsmittel vorzugsweise destillativ entfernt und das Reaktionsprodukt gleichfalls destillativ aufgearbeitet, wobei eine Vakuumdestillation, vorzugsweise im Bereich von 1 bis 20 Torr vorteilhaft ist.

Weiterhin ist es erfindungsgemäß bevorzugt, die hydantoinhaltige Diisocyanate der Formel (I) mit weiteren Isocyanaten wie Polyisocyanate und Diisocyanate, vorzugsweise Diisocyanaten, wie sie der Auflistung der Polyisocyanaten (a) zu entnehmen sind, zu mischen. Besonders bevorzugt sind jedoch Mischungen mit Diisocyanaten, im folgenden als Standarddiisocyanate bezeichnet, wobei Diarylalkyl-Diisocyanate, vorzugsweise Diphenylmethan-Diisocyanat und insbesondere bevorzugt 4,4-Diphenylmethan-Diisocyanat (MDI) und polymeres Diarylalkyl-Diisocyanat, vorzugsweise Diphenylmethan-Diisocyanat und besonders bevorzugt 4,4'-Diphenymethan-Diisocyanat (Polymer-MDI) bevorzugt sind.

Vorzugsweise wird erfindungsgemäß ein Gemisch aus einem Zweikern-MDI mit höherkernigen polymeren Diisocyanaten eingesetzt. Vorzugsweise weist dieses Gemisch einen viskositätsbereich von 50 bis 3000 und bevorzugt von 90 bis 2500 mPa · s/25° C auf. Weiterhin ist es bevorzugt, daß dieses Gemisch einen NCO-Gehalt von 30 - 34 Gew.-% enthält. Darüber hinaus ist es erfindungsgemäß bevorzugt, daß der Mono-MDI-Gehalt 10 bis 80 und bevorzugt 20 bis 60 Volumenteile des gesamten MDI-Gemisches beträgt.

Die erfindungsgemäßen hydantoinhaltigen Diisocyanate der Formel (I), sowie deren Mischungen mit anderen Isocyanaten lassen sich für die Herstellung von Polyurethanen verwenden, die wiederum für die Herstellung von Fasern, Folien, Schäumen, Formmassen, Beschichtungen und Lacken verwendet werden können.

Besonders bevorzugt wird aus mindestens einem hydantoinhaltigen Diisocyanat der Formel (I) oder mindestens einer der vorgenannten Mischungen zusammen mit mindestens einem Polyol ein Polyurethan-Hartschaum hergestellt. Die Polyurethane und insbesondere die Polyurethan-Hartschäume werden nach guter Durchmischung der Komponenten in einem Temperaturbereich von 10 bis 70, bevorzugt 20 bis 60 und besonders bevorzugt 35 bis 55 °C hergestellt. Besonders bevorzugt erfolgt der Herstellung bei Polyurethan-Hartschäumen in einem Formwerkzeug, indem die Form des bei der Polyurethanbildung entstehenden Hartschaumkörpers als Negativ ausgebildet ist. Die aus den Polyurethan-Hartschäumen gewonnenen Formkörper weisen vorzugsweise eine Dichte von 50 bis 90, bevorzugt 60 bis 80 und besonders bevorzugt 65 bis 75 kg/m³ auf. Die Entformungszeit beträgt vorzugsweise 10 bis 60, bevorzugt 20 bis 40 und besonders bevorzugt 25 bis 35 Minuten.

Das erfindungsgemäße hydantoinhaltige Diisocyanat der Formel (I), insbesondere die Diisocyanate der Formel (I), bei denen der Rest R³ ein C₁-C₁₂-, bevorzugt C₄-C₈- und besonders bevorzugt C₅-C₆ -Alkyl ist, das vorzugsweise unverzweigt ist, können mit entsprechenden Hydroxylgruppen tragenden Acrylatharzen zu Lacken umgesetzt werden. Als für die Lacksynthese besonders geeignet hat sich ein hydantoinhaltiges Diisocyanat der Formel (I) erwiesen, daß von 1,6-Hexamethylen-diisocyanat als Diisocyanat der Formel (III) ausgeht. Als hydroxylgruppenhaltige Acrylatharze kommen insbesondere welche mit einem Gewichtsmittel des Molekulargewichts in einem Bereich von 2000 bis 20.000, bevorzugt 3000 bis 10.000 und besonders bevorzugt 5000 bis 7000 g/mol in Betracht. Ferner weisen die geeigneten Acrylatharze vorzugsweise eine OH Zahl in Bereiche von 50 bis 200, bevorzugt 70 bis 150 und besonders bevorzugt 80 bis 140 auf.

In den erfindungsgemäßen Polyurethanen, Polyurethanhartschäumen und Polyurethanlakken, vorzugsweise in den Polyurethanlacken können Pigmente, vorzugsweise TiO₂ als Weißpigment, eingesetzt werden.

Das erfindungsgemäße Diisocyanat der Formel (I) führt weiterhin dazu, daß die Polyisocyanatkomponente (a) mit diesem dünnflüssiger als ohne ist. Daher kann vorzugsweise bei Lacken, vorzugsweise mit einem hydroxylgruppenhaltigen Acrylatharzen, die als einen Bestandteil eine Mischung aus dem Diisocyanat der Formel (I) und der Komponente (a) ausweisen, mit einer geringeren Lösungsmittelmenge die gleiche Viskosität erreicht werden, wie ohne das Diisocyanat der Formel (I). Weiterhin läßt sich durch die Anwesenheit des Diisocyanats der Formel (I) die Elastizität von den vorgenannten Lacken gegenüber vergleichbaren Lacken herkömmlicher Zusammensetzung verbessern.

Nachfolgend werden die Bestandteile bzw. Komponenten (a) bis (g) beispielhaft erläutert.

### Polyisocyanate (a)

Als organische Polyisocyanate kommen vorzugsweise die an sich bekannten aliphatischen, cycloaliphatischen und besonders bevorzugt aromatischen mehrwertigen Isocyanate in Frage. Besonders bevorzugt sind in einer erfindungsgemäßen Ausführungsform Polyisocyanate mit zwei Isocyanatgruppen, wenn derartige Polyisocyanate nicht vernetzend wirken sollen. Ist jedoch die vernetzende Wirkung der Polyisocyanate bevorzugt, werden dann in einer erfindungsgemäßen Ausführungsform Polyisocyanate mit mehr als zwei Isocyanatgruppen verwandt.

Besonders bevorzugte organische Polyisocyanate (a) sind beispielsweise Alkylendiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylendiisocyanat-1,5, Tetramethylendiisocyanat-1,4 und vorzugsweise Hexamethylendiisocyanat-1,6; cycloaliphatische Diisocyanate, wie Cyclohexan-1,3- und -1,4-diisocyanat sowie beliebige Gemische dieser Isomeren, 1-Isocyanato-3,3,5-trimethyl-5-isocyanatomethylcyclohexan (IPDI), 2,4- und 2,6-Hexahydrotoluylendiisocyanat sowie die entsprechenden Isomerengemische, 4,4'-, 2,2'- und 2,4'-Dicyclohexylmethandiisocyanat sowie die entsprechenden Isomerengemische, und vorzugsweise aromatische Di- und Polyisocyanate, wie beispielsweise 2,4- und 2,6-Toluylendiisocyanat und die entsprechenden Isomerengemische, 4,4'-, 2,4'- und 2,2'-Diphenylmethandiisocyanat und die entsprechenden Isomerengemische, Mischungen aus 4,4'- und 2,2'-Diphenylmethandiisocyanaten, Polyphenylpolymethylenpolyisocyanate, Mischungen aus 4,4'-, 2,4'- und 2,2'-Diphenylmethandiisocyanaten und Polyphenylpolymethylenpolyisocyanaten, beispielsweise Roh-MDI und Mischungen aus Roh-MDI und Toluylendiisocyanaten. Die organischen Di- und Polyisocyanate können einzeln oder in Form ihrer Mischungen eingesetzt werden.

Häufig werden als organische Polyisocyanate (a) auch sogenannte modifizierte mehrwertige Isocyanate, d.h. Produkte, die durch chemische Umsetzung organischer Di- und/oder Polyisocyanate erhalten werden, verwendet. Beispielhafte Isocyanate dieser Art sind Ester-, Harnstoff-, Biuret-, Carbodiimid-, Isocyanurat-, und/oder Urethangruppen enthaltende Di- und/oder Polyisocyanate. Im einzelnen kommen beispielsweise in Betracht: Urethangruppen enthaltende organische, vorzugsweise aromatische Polyisocyanate mit einem NCO-Gehalt von 33,6 bis 15 Gew.-%, vorzugsweise von 31 bis 21 Gew.-%, bezogen auf das Gesamtgewicht; beispielsweise mit niedermolekularen Diolen, Triolen, Dialkylenglycolen, Trialkylenglycolen oder Polyoxyalkylenglycolen mit Molekulargewichten bis 6.000, insbesondere mit Molekulargewichten bis 1.500, modifiziertes 4,4'-Diphenylmethandiisocyanat, modifizierte 4,4'- und 2,4'-Diphenylmethandiisocyanat-Mischungen oder modifiziertes Roh-MDI, wobei Di- bzw. Polyoxyalkylenglycole einzeln oder als Gemische eingesetzt werden können. Beispielhaft genannt seien: Diethylen-, Dipropylenglycol, Polyoxyethylen-, Polyoxypropylen- und Polyoxypropylenpolyethylendiole, -triole und/oder -tetrole. Erfihdungsgemäß vorzugsweise geeignet sind auch NCO-Gruppen enthaltende Prepolymere mit NCO-Gehalten von 25 bis 3,5 Gew.-%, vorzugsweise von 21 bis 14 Gew.-%, bezogen auf das Gesamtgewicht des Prepolymers, hergestellt aus den nachfolgend beschriebenen Polyester- und/oder vorzugsweise Polyetherpolyolen und 4,4'-Diphenylmethandiisocyanat, Mischungen aus 2,4'- und 4,4'-Diphenylmethandiisocyanat, 2,4- und/oder 2,6-Toluylendiisocyanaten oder Roh-MDI. Weiterhin bevorzugt sind flüssige, Carbodiimidgruppen und/oder Isocyanuratringe enthaltende Polyisocyanate mit einem NCO-Gehalt von 33,6 bis 15 Gew.-%, vorzugsweise 31 bis 21 Gew.-%, bezogen auf das Gesamtgewicht des Polyurethans, beispielsweise auf Basis von 4,4'-, 2,4'- und/oder 2,2'-Diphenylmethandiisocyanat und/oder 2,4- und/oder 2,6-Toluylendiisocyanat.

Die modifizierten Polyisocyanate können vorzugsweise miteinander oder mit unmodifizierten organischen Polyisocyanaten, beispielsweise 2,4'-, 4,4'-Diphenylmethandiisocyanat, Roh-MDI, 2,4- und/oder 2,6-Toluylendiisocyanat gemischt werden.

Besonders bevorzugt sind die folgenden organischen Polyisocyanate, die sich besonders bei der Herstellung von zelligen Elastomeren bewährt haben:

NCO-Gruppen enthaltende Prepolymere mit einem NCO-Gehalt von 25 bis 9 Gew.-% bezogen auf das Prepolymer, insbesondere auf Basis von Polyether- oder Polyesterpolyolen und einem oder mehreren Diphenylmethandiisocyanatisomeren, vorteilhafterweise 4,4'-Diphenylmethandiisocyanat und/oder modifizierte Urethangruppen enthaltende organische Polyisocyanate mit einem NCO-Gehalt von 33,6 bis 15 Gew.-%, insbesondere auf Basis von 4,4'-Diphenylmethandiisocyanat oder Diphenylmethandiisocyanatisomeren-Gemischen.

Zur Herstellung von flexiblen Polyurethanschaumstoffen als Untergruppe der zelligen Elastomere haben sich bewährt:

Mischungen aus 2,4- und 2,6-Toluylendiisocyanaten, Mischungen aus Toluylendiisocyanaten und Polyphenylpolymethylenpolyisocyanat oder insbesondere Mischungen aus den vorgenannten Prepolymeren auf Basis von Diphenylmethandiisocyanatisomeren und Roh-MDI, vorzugsweise Polyphenylpolymethylenpolyisocyanat, mit einem Diphenylmethandiisocyanat-Isomerengehalt von 30 bis 80 Gew.-%.

### Verbindungen mit mindestens zwei reaktiven Wasserstoffen (b)

Als organische Verbindungen mit mindestens zwei reaktiven Wasserstoffen (b) werden vorzugsweise die oben näher charakterisierten Verbindungen, gegebenenfalls im Gemisch mit weiteren mit einer Funktionalität von 2 bis 8, vorzugsweise 2 bis 4, und einem Molekulargewicht von 300 bis 10.000, vorzugsweise 1.000 bis 6.000, verwendet. Besonders bevorzugt sind beispielsweise Polyetherpolyamine und/oder vorzugsweise Polyole, ausgewählt aus der Gruppe der Polyetherpolyole, Polyesterpolyole, Polythioetherpolyole, Polyesteramide, hydroxygruppenhaltige Polyacetate und hydroxygruppenhaltige aliphatische Polycarbonate oder Mischungen aus mindestens zwei der genannten Polyole. Vorzugsweise Anwendung finden Polyesterpolyole und/oder Polyetherpolyole.

Geeignete Polyesterpolyole können beispielsweise aus organischen Dicarbonsäuren mit 2 bis 12 Kohlenstoffatomen, vorzugsweise aliphatischen Dicarbonsäuren mit 4 bis 6 Kohlenstoffatomen, mehrwertigen Alkoholen, vorzugsweise Diolen, mit 2 bis 12 Kohlenstoffatomen, vorzugsweise 2 bis 6 Kohlenstoffatomen, hergestellt werden. Als Dicarbonsäuren kommen vorzugsweise in Betracht: Bernsteinsäure, Glutarsäure, Adipinsäure, Korksäure, Azelainsäure, Sebacinsäure, Decandicarbonsäure, Maleinsäure, Fumarsäure, Phthalsäure, Isophthalsäure und Terephthalsäure. Die Dicarbonsäuren können dabei sowohl einzeln als auch im Gemisch verwendet werden. Anstelle der freien Dicarbonsäuren können auch die entsprechenden Dicarbonsäurederivate, beispielsweise Dicarbonsäureester von Alkoholen mit 1 bis 4 Kohlenstoffatomen oder Dicarbonsäureanhydride eingesetzt werden. Vorzugsweise verwendet werden Dicarbonsäuregemische aus Bernstein-, Glutar- und Adipinsäure und insbesondere Adipinsäure. Beispiele für zwei- und mehrwertige Alkohole, insbesondere Diole, sind: Ethandiol, Diethylenglycol, 1,2- bzw. 1,3-Propandiol, Dipropylenglycol, 1,4-Butandiol, 1,5-Pentandiol, 1,6-Hexandiol, 1,10-Decandiol, Glycerin und Trimethylolpropan. Vorzugsweise verwendet werden Ethandiol, Diethylenglycol, 1,4-Butandiol, 1,5-Pentandiol und 1,6-Hexandiol. In dem erfindungsgemäßen Verfahren zur Herstellung der Polyurethane können weiterhin eingesetzt werden: Polyesterpolyole aus Lactonen, beispielsweise ε-Caprolacton oder Hydroxycarbonsäuren, beispielsweise ω-Hydroxycarbonsäuren.

Zur Herstellung der Polyesterpolyole können die organischen, beispielsweise aromatischen und vorzugsweise aliphatischen Polycarbonsäuren und/oder Derivate und mehrwertigen Alkohole katalysatorfrei oder vorzugsweise in Gegenwart von Veresterungskatalysatoren polykondensiert werden. Die Polykondensation wird vorzugsweise in einer Atmosphäre aus Inertgas, beispielsweise Stickstoff, Kohlenmonoxid, Helium, Argon und anderen, in der Schmelze bei Temperaturen von 150 bis 200°C, vorzugsweise 180 bis 220°C, gegebenenfalls unter vermindertem Druck, bis zu einer gewünschten Säurezahl durchgeführt. Die resultierende Säurezahl beträgt vorteilhafterweise < 10, vorzugsweise < 2. Nach einer bevorzugten Ausführungsform wird als Veresterungsgemisch bei den oben genannten Temperaturen bis zu einer Säurezahl von 80 bis 30, vorzugsweise 40 bis 30, unter Normaldruck und anschließend unter einem Druck von < 500 mbar, vorzugsweise 50 bis 150 mbar, polykondensiert. Als Veresterungskatalysatoren kommen beispielsweise Eisen-, Cadmium-, Cobalt-, Blei-, Zink-, Antimon-, Magnesium-, Titan- und Zinn-Katalysatoren in Form von Metallen, Metalloxiden oder Metallsalzen in Betracht.

Die Polykondensation kann jedoch auch in Gegenwart von Verdünnungs- und/oder Schleppmitteln, wie beispielsweise Benzol, Toluol, Xylol oder Chlorbenzol zur azeotropen Abdestillation des Kondensationswassers durchgeführt werden. Zur Herstellung der Polyesterpolyole werden die organischen Polycarbonsäuren und/oder Derivate und mehrwertige Alkohole vorteilhafterweise im Molverhältnis von 1:1 bis 1,8, vorzugsweise 1:1,05 bis 1,2, polykondensiert.

Die erhaltenen Polyesterpolyole besitzen vorzugsweise eine Funktionalität von 2 bis 4, insbesondere 2 bis 3, und ein Molekulargewicht von 480 bis 3.000, vorzugsweise 1.000 bis 3.000.

Als Polyole werden jedoch vorzugsweise Polyetherpolyole verwendet, die nach bekannten Verfahren, beispielsweise durch anionische Polymerisation mit Alkalihydroxiden, wie beispielsweise Natrium- oder Kaliumhydroxid oder Alkalialkoholaten, wie beispielsweise Natriummethylat, Natrium- oder Kaliumethylat oder Kaliumisopropylat, als Katalysatoren und unter Zusatz mindestens eines Startermoleküls, das 2 bis 8, vorzugsweise 2 bis 4, reaktive Wasserstoffatome gebunden enthält, hergestellt werden.

Ferner kann die Herstellung durch kationische Polymerisation mit Lewissäuren, wie Antimonpentachlorid, Borfluorid-etherat u.a. oder Bleicherde, als Katalysatoren aus einem oder mehreren Alkylenoxiden mit 2 bis 4 Kohlenstoffatomen tragenden Alkylresten durchgeführt werden.

Geeignete Alkylenoxide sind beispielsweise 1,3-Propylenoxid, 1,2- bzw. 2,3-Butylenoxid, Styroloxid, THF und vorzugsweise Ethylenoxid und 1,2-Propylenoxid. Die Alkylenoxide können einzeln, alternierend nacheinander oder als Mischungen verwendet werden.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono- N,N- und N,N'-dialkylsubstituierte Diamine mit 1 bis 2 Kohlenstoffatomen im Alkylrest, wie gegebenenfalls mono- und dialkylsubstituiertes Ethylendiamin, Diethylentriamin, Triethylentetraamin, 1,3-Proyplendiamin, 1,3- bzw. 1,4-Butylendiamin, 1,2-, 1,3-, 1,4-, 1,5- und 1,6-Hexamethylendiamin, Phenylendiamin, 2,3-, 2,4- und 2,6-Toluylendiamin und 4,4'-, 2,4'- und 2,2'-Diaminodiphenylmethan.

Als Startermoleküle kommen ferner vorzugsweise in Betracht: Alkanolamine, beispielsweise Ethanolamin, N-Methyl- und N-Ethylethanolamin, Dialkanolamine, wie beispielsweise Diethanolamin, N-Methyl- und N-Ethyldiethanolamin, und Trialkanolamine, wie beispielsweise Triethanolamin sowie Ammoniak. Vorzugsweise verwendet werden mehrwertige, insbesondere zwei- und/oder dreiwertige Alkohole, beispielsweise Ethandiol, Propandiol-1,2 und -2,3, Diethylenglycol, Dipropylenglycol, Butandiol-1,4, Hexandiol-1,6, Glycerin, Trimethylolpropan und Pentaerythrit oder Zucker, vorzugsweise Rübenzucker.

Die Polyetherpolyole, vorzugsweise Polyoxypropylen- und Polyoxypropylenpolyoxyethylenpolyole, besitzen eine Funktionalität von vorzugsweise 2 bis 8 und ein Molekulargewicht von 300 bis 10.000, vorzugsweise 1.000 bis 6.000 und insbesondere bevorzugt von 1.500 bis 5.000. Geeignete Poly- oxytetramethylenglycole besitzen ein Molekulargewicht von bis zu ungefähr 3.500.

Weiterhin eignen sich als Polyetherpolyole insbesondere polymermodifizierte Polyetherpolyole, vorzugsweise Pfropfpolyetherpolyole, insbesondere solche auf Styrol- und/oder Acrylnitrilbasis. Diese lassen sich durch *In-Situ*-Polymerisation von Acrylnitril, Styrol oder vorzugsweise Mischungen aus Styrol und Acrylnitril, beispielsweise im Gewichtsverhältnis von 90 : 10 bis 10 : 90, vorzugsweise 70 : 30 bis 30 : 70, in zweckmäßiger Weise mit den vorgenannten Polyetherpolyolen analog den Angaben der deutschen Patentschriften 11 11 394, 12 22 669 (US 3,304,273, 3,383,351, 3,523,039), 1 152 536 (GB 1 040 452) und 1 152 537 (GB 9 876 618) herstellen. Ferner eignen sich Polyetherpolyoldispersionen, die als disperse Phasen, üblicherweise in einer Menge von 1 bis 50 Gew.-%, vorzugsweise 2 bis 25 Gew.-%, z.B. Polyharnstoffe, Polyhydrazide, tert.-Aminogruppen gebunden enthaltende Polyurethane und/oder Melamin enthalten. Solche Dispersionen werden beispielsweise in der EP-B-011 752 (US 4,304,708), US-4,374,209 und DE-A-32 31 497 beschrieben.

Die Polyetherpolyole können ebenso wie die Polyesterpolyole einzeln oder in Form von Mischungen verwendet werden. Ferner können sie mit den Pfropfpolyetherpolyolen oder Polyesterpolyolen sowie den hydroxygruppenhaltigen Polyesteramiden, Polyacetalen, Polycarbonaten und/oder Polyetherpolyaminen gemischt werden.

Als hydroxygruppenhaltige Polyacetale kommen beispielsweise die aus Glycolen, wie Diethylenglycol, Triethylenglycol, 4,4'-Dihydroxyethylendiphenyldimethylmethan, Hexandiol und Formaldehyd, herstellbaren Verbindungen in Frage. Auch durch Polymerisation zyklischer Acetale lassen sich geeignete Polyacetale herstellen.

Als Hydroxygruppen aufweisende Polycarbonate kommen insbesondere solche in Betracht, die beispielsweise durch Umsetzung von Diolen, wie Propandiol-1,3, Butandiol-1,4 und/oder Hexandiol-1,6, Diethylenglycol, Triethylenglycol oder Tetraethylenglycol mit Diarylcarbonaten, beispielsweise Diphenylcarbonat oder Phosgen, hergestellt werden können.

Zu den bevorzugten Polyesteramiden zählen beispielsweise die aus mehrwertigen, gesättigten und/oder ungesättigten Carbonsäuren, beispielsweise deren Anhydriden oder mehrwertigen gesättigten und/oder ungesättigten Aminoalkoholen oder Mischungen aus mehrwertigen Alkoholen und Aminoalkoholen und/oder Polyaminen gewonnenen, vorwiegend linearen Kondensate.

Bevorzugte erfindungsgemäß geeignete Polyetherpolyamine können aus den oben genannten Polyetherpolyolen nach bekannten Verfahren hergestellt werden. Beispielhaft genannt seien die Cyanoalkylierung von Polyoxyalkylenpolyolen und anschließende Hydrierung des gebildeten Nitrils (US 3,267,050) oder die teilweise oder vollständige Aminierung von Polyoxyalkylenpolyolen mit Aminen oder Ammoniak in Gegenwart von Wasserstoff und Katalysatoren (DE 12 15 373).

Erfindungsgemäß besonders bevorzugt sind 2- bis 3-funktionelle Propylen- und Ethylenoxid aufweisende Polyetherole, in denen vorzugsweise 10 bis 50, bevorzugt 15 bis 30 und besonders bevorzugt 20 bis 25 Gew.-% Ethylenoxid eingebaut sind und die ferner eine OH-Zahl im Bereich von 10 bis 50, bevorzugt 15 bis 40 und besonders bevorzugt 20 bis 30 besitzen.

### Kettenverlängerungs- und/oder Vernetzungsmittel (c)

Die Polyurethane können ohne oder unter Mitverwendung von Kettenverlängerungs- und/oder Vernetzungsmitteln hergestellt werden. Zur Modifizierung der mechanischen Eigenschaften, beispielsweise der Härte, kann sich jedoch der Zusatz von Kettenverlängerungsmitteln, Vernetzungsmitteln oder gegebenenfalls auch Gemischen davon als vorteilhaft erweisen. Die Kettenverlängerungs- oder Vernetzungsmittel stellen, sofern sie mindestens zwei reaktive Wasserstoffatome besitzen, eine Untergruppe der Verbindungen mit mindestens zwei reaktiven Wasserstoffen (Komponente (a)) dar.

Als Kettenverlängerungs- und/oder Vernetzungsmittel werden Diole und/oder Triole mit Molekulargewichten <400, vorzugsweise 60 bis < 300 eingesetzt.
In Betracht kommen beispielsweise aliphatische, cycloaliphatische und/oder araliphatische Diole mit 2 bis 14, vorzugsweise 2 bis 10 Kohlenstoffatomen, beispielsweise Ethylenglycol, Propandiol-1,3, Decandiol-1,10, o-, m-, p-Dihydroxycyclohexan, Diethylenglycol, Dipropylenglycol und vorzugsweise Butandiol-1,4, Hexandiol-1,6 und Bis-(2-hydroxyethyl)-hydrochinon, Triole, beispielsweise 1,2,3-Trihydroxycyclohexan, Glycerin und Trimethylolpropan und niedermolekulare hydroxygruppenhaltige Polyalkylenoxide auf Basis von Ethylen- und/oder 1,2-Propylenoxid und den vorgenannten Diolen und/oder Triolen als Startermoleküle.

Zur Herstellung von zelligen Elastomerformkörpern und Integralschaumstoffen können außer den oben genannten Diolen und/oder Triolen oder im Gemisch mit diesen als Kettenverlängerungs und/oder Vernetzungsmittel (b) auch sekundäre aromatische Diamine, 3,3'-Di- und/oder 3,3'-, 5,5'-tetraalkylsubstituierte Diaminodiphenylmethane Anwendung finden.

Als sekundäre aromatische Diamine seien vorzugsweise genannt: N,N'-dialkylsubstituierte aromatische Diamine mit 1 bis 20, vorzugsweise 1 bis 4 Kohlenstoffatomen im N-Alkylrest, wie N,N'-Di-sek.-phenyl-, N,N'-Di-sek.-hexyl-, N,N'-Di-sek.-decyl-, N,N'-Dicyclohexyl-p- bzw. -m-phenylendiamin, N,N'-Dimethyl-, N,N'-Diethyl-, N,N'-Diisopropyl-, N,N'-Di-sek.-butyl-, N,N'-Dicyclohexyl-4,4'-diaminophenylmethan und N,N'-Di-sek.-butyl-benzidin. Diese können ggf. am aromatischen Kern durch Alkylreste substituiert sein.

### Katalysatoren (d)

Als Katalysator (d) zur Herstellung von Polyurethanen werden insbesondere Verbindungen verwendet, die die Reaktion der reaktiven Wasserstoffatome, insbesondere Hydroxylgruppen enthaltenden Verbindungen der Komponenten (b) und gegebenenfalls (c) mit den organischen, gegebenenfalls modifizierten Polyisocyanaten (a) stark beschleunigen. In Betracht kommen organische Metallverbindungen, vorzugsweise organische Zinnverbindungen, wie Zinn-(II)-Salze von organischen Carbonsäuren, beispielsweise Zinn-(II)-acetat, Zinn-(II)-octoat, Zinn-(II)-ethylhexoat und Zinn-(II)-laurat und die Dialkylzinn-(IV)-Salze von organischen Carbonsäuren, beispielsweise Dibutylzinndiacetat, Dibutylzinn-dilaurat, Dibutylzinnmaleat und Dioctylzinndiacetat. Die organischen Metallverbindun-gen werden alleine oder vorzugsweise in Kombination mit stark basischen Aminen eingesetzt. Genannt seien beispielsweise Amidine, wie 2,3-Dimethyl-3,4,5,6-tetrahydropyrimidin, tertiäre Amine, wie Triethylamin, Tributylamin, Dimethylbenzylamin, N-Methyl-, N-Ethyl-, N-Cyclohexylmorpholin, N,N,N',N'-Tetramethylethylendiamin, N,N,N',N'-Tetramethylbutandiamin, N,N,N',N'-Tetramethylhexandiamin-1,6, Pentamethyldiethylentriamin, Tetramethyldiaminoethylether, Bis-(dimethylaminopropyl)-harnstoff, Dimethylpiperazin, 1,2-Dimethylimidazol, 1-Azabicyclo-(3,3,0)-octan und vorzugsweise 1,4-Diazabicyclo-(2,2,2)-octan, und Alkanolverbindungen, wie Triethanolamin, Triisopropanolamin, N-Methyl- und N-Ethyldiethanolamin und Dimethylethanolamin.

Als Katalysatoren sind weiterhin bevorzugt: Tris-(dialkylaminoalkyl)-s-hexahydrotriazine, insbesondere Tris-(N,N-dimethylaminopropyl)-s-hexahydrotriazin, Tetraalkylammoniumhydroxide, wie Tetramethylammoniumhydroxid, Alkalihydroxid, wie Natriumhydroxid, und Alkalialkoholate, wie Natriummethylat und Kaliumisopropylat, sowie Alkalisalze von langkettigen Fettsäuren mit 10 bis 20 C-Atomen und gegebenenfalls seitenständigen OH-Gruppen. Vorzugsweise werden 0,001 bis 5 Gew.-%, insbesondere 0,05 bis 2 Gew.-%, Katalysator bzw. Katalysatorkombinationen, bezogen auf das Gewicht der Komponente (b), verwendet.

### Treibmittel (e)

Zur Herstellung von Polyurethan-Schaumstoffen wird als chemisches Treibmittel (e) Wasser verwendet, das mit Isocyanatgruppen unter Bildung von Amingruppen und Kohlendioxid, dem wirklichen Treibgas, reagiert. Die Wassermengen, die zweckmäßigerweise eingesetzt werden, betragen 0,1 bis 5 Gew.-Teile, vorzugsweise 1,5 bis 3,5 Gew.-Teile und besonders bevorzugt 2,0 bis 3,0 Gew.-Teile, bezogen auf 100 Gew.-Teile der Polyhydroxyverbindung (b) bzw., Mischungen aus organischer Verbindung mit mindestens zwei reaktiven Wasserstoffen, vorzugsweise höhermolekularen Polyhydroxyverbindungen (b) mit Kettenverlängerungs- und/oder Vernetzungsmitteln (c).

Im Gemisch mit Wasser können als Treibmittel (e) auch physikalisch wirkende Treibmittel eingesetzt werden. Geeignet sind insbesondere Flüssigkeiten, welche gegenüber den organischen, gegebenenfalls modifizierten Polyisocyanaten (a) inert sind und Siedepunkt unter 100°C, vorzugsweise unter 50°C und besonders bevorzugt zwischen -50 und 30°C bei Atmosphärendruck ausweisen, so daß sie unter dem Einfluß der exothermen Polyadditionsreaktion verdampfen. Beispiele derartiger Flüssigkeiten sind Kohlenwasserstoffe, beispielsweise iso-Pentan, vorzugsweise technische Gemische aus n- und iso-Pentan, n- und iso-Butan, n- und iso-Propan, Cycloalkane, wie beispielsweise Cyclohexan und Cyclopentan, Ether, beispielsweise Furan, Dimethylether und Dieethylether, Ketone, beispielsweise Aceton und Methylethylketon, Carbonsäurealkylester, beispielsweise Methylformiat, Dimethyloxalat und Ethylacetat und halogenierte Kohlenwasserstoffe, beispielsweise Methylenchlorid, Dichlormonofluormethan, Difluormethan, Difluorchlormethan, Trifluormethan, Difluorethan, Tetrafluor-ethan, Heptafluorpropan, 1-Chlor-2,2-difluorethan, 1-Chlor-1,1-difluorethan und 1-Chlor-1,2-difluorethan. Auch Gemische dieser niedrigsiedenden Flüssigkeiten, beispielsweise aus Difluormethan und 1-Chlor-1,1-difluorethan und/oder mit anderen halogensubstituierten oder unsubstituierten Kohlenwasserstoffen können verwendet werden. Ein erfindungsgemäß ferner bevorzugtes Treibmittel ist CO₂ für sich oder im Gemisch mit anderen Treibmitteln, vorzugsweise Wasser.

Die Menge an physikalisch wirkenden Treibmitteln kann in Abhängigkeit von der gewünschten Schaumstoffdichte auf einfache Weise ermittelt werden und beträgt 0 bis 25 Gew.-Teile, vorzugsweise 1 bis 25 Gew.-Teile und besonders bevorzugt 2 bis 15 Gew.-Teile pro 100 Gew.-Teile der Polyhydroxylverbindungen (b). Gegebenenfalls kann es zweckmäßig sein, die gegebenenfalls modifizierten Polyisocyanate (a) mit dem inerten physikalisch wirkenden Treibmittel zu mischen und dadurch die Viskosität zu verringern.

### Flammschutzmittel (f)

Geeignete Flammschutzmittel (f) neben dem Phophat der Formel (IV) sind beispielsweise Trikresylphosphat, Tris-(2-chlorethyl)phosphat, Tris-(2-chlorpropyl)phosphat, Tetrakis-(2-chlorethyl)-ethylendiphosphat, Dimethylmethanphosphonat, Diethanolaminomethylphosphonsäurediethylester sowie handelsübliche halogenierte Flammschutzpolyole.

Im allgemeinen hat es sich als zweckmäßig erwiesen 5 bis 50, Gew. -Teile, vorzugsweise 5 bis 25 Gew.-Teile, der genannten Flammschutzmittel für jeweils 100 Gew.-Teile der Komponenten (a) bis (e) zu verwenden.

Nähere Angaben über die oben genannten anderen üblichen Hilfs- und Zusatzstoffe sind in der Fachliteratur, beispielsweise in der Monographie von J.H. Saunders und K.C. Frisch *"High Polymers",* Bd. XVI, *Polyurethanes,* Teil 1 u. Teil 2, Verlag Interscience Publishers, 1962 bzw. 1964, oder dem *Kunststoff-Handbuch, Polyurethane,* Bd.VII, Hanser-Verlag, München/Wien, 1. u. 2. Aufl., 1966 und 1983, zu entnehmen.

Zur Herstellung der erfindungsgemäßen Polyurethane werden die organischen Polyisocyanate (a) organische Verbindungen mit mindestens zwei reaktiven Wasserstoffatomen (b) und gegebenenfalls Kettenverlängerungs- und/oder vernetzungsmittel (c) in solchen Mengen zur Umsetzung gebracht, daß das Äquivalenzverhältnis der NCO-Gruppen der Polyisocyanate (a) zu der Summe der reaktiven Wasserstoffatome der Komponente (b) und gegebenenfalls (c) sowie bei der Verwendung von Wasser als Treibmittel auch des Wassers 0,85 bis 1,25 : 1, vorzugsweise 0,95 bis 1,15 : 1 und insbesondere 1,05 : 1, beträgt. Falls die Polyurethane zumindest teilweise Isocyanuratgruppen gebunden enthalten, wird üblicherweise ein Verhältnis von NCO-Gruppen der Polyisocyanate (a) zur Summe der reaktiven Wasserstoffatome der Komponente (b) und gegebenenfalls (c) von 1,5 bis 20 : 1, vorzugsweise 1,5 bis 8 : 1, angewandt.

### Hilfsmittel und/oder Zusatzstoffe (g)

Der Reaktionsmischung zur Herstellung der Formkörper mit verdichteter Randzone und zelligem Kern können gegebenenfalls Hilfsmittel und/oder Zusatzstoffe (g) einverleibt werden. Genannt seien beispielsweise oberflächenaktive Substanzen, Schaumbildungsstabilisatoren, Zellregler, Füllstoffe, Farbstoffe, Pigmente, gegebenenfalls Flammschutzmittel, Hydrolyseschutzmittel, fungistatisch und bakteriostatisch wirkende Substanzen. Als oberflächenaktive Substanzen kommen beispielsweise Verbindungen in Betracht, welche zur Unterstützung der Homogenisierung der Ausgangsstoffe dienen und gegebenenfalls auch geeignet sind, die Zellstruktur der Grundstoffe zu regulieren. Als bevorzugte oberflächenaktive Substanzen seien beispielsweise Emulgatoren, wie die des Natriumsalzes der Ricinusölsulfate oder von Fettsäuren, sowie Salzen von Fettsäuren mit Aminen, beispielsweise ölsaures Diethyl- amin, stearinsaures Diethanolamin, ricinolsaures Diethanolamin, Salze von Sulfonsäuren, beispielsweise Alkali- oder Ammoniumsalze von Dodecylbenzol- oder Dinaphthylmethandisulfonsäure oder Ricinolsäure; Schaumstabilisatoren, wie Siloxanoxalkylen-Mischpolymerisate und andere Organopolysiloxane, oxethylierte Alkylphenole, oxethylierte Fettalkohole, Paraffinöle, Ricinusöl- bzw. ricinolsaure Ester, Türkischrotöl, Erdnußöl und Zellregler, wie Paraffine, Fettalkohole, Dimethylpolysiloxane genannt. Zur Verbesserung der Emulgierwirtung, der Zellstruktur und/oder Stabilisierung des Schaumes eignen sich ferner oligomere Acrylate mit Polyoxalkylen- und Fluoralkanresten als Seitengruppen. Die oberflächenaktiven Substanzen werden üblicherweise in Mengen von 0,01 bis 5 Gew.-Teilen, bezogen auf 100 Gew.-Teile der Komponente (b) bis (f) angewandt.

Als geeignete Trennmittel seien vorzugsweise genannt: Umsetzungsprodukte von Fettsäureestern mit Polyisocyanaten, Salze aus Aminogruppen enthaltenden Polysiloxanen und Fettsäuren, Salze aus gesättigten oder ungesättigten (cyclo)aliphatischen Carbonsäuren mit mindestens 8 C-Atomen und tertiären Aminen sowie insbesondere innere Trennmittel, beispielsweise Carbonsäure- ester und/oder -amide, gemäß EP-A-153 639 oder DE-A-36 07 447.

Als Füllstoffe, insbesondere verstärkend wirkende Füllstoffe, sind die an sich bekannten, üblichen organischen und anorganischen Füllstoffe, Verstärkungsmittel, Beschwerungsmittel, Mittel zur Verbesserung des Abriebverhaltens in Anstrichfarben, Beschichtungsmittel usw. zu verstehen.

Im einzelnen seien bevorzugte beispielhaft genannt:

Anorganische Füllstoffe wie silicalitische Mineralien, beispielsweise Schichtsilicate, wie Antigorit, Serpentit, Hornblende, Amphibole, Chrysolit, Talkum; Metalloxide, wie Kaolin, Aluminiumoxide, Titanoxide und Eisenoxide, Metallsalze, wie Kreide, Schwerspat und anorganische Pigmente, wie Cad-miumsulfid, Zinksulfid, sowie Glas u.a. Vorzugsweise verwendet werden Kaolin (China Clay), Aluminiumsilicat und Copräzipitate aus Bariumsulfat und Aluminiumsilicat sowie natürliche und synthetische faserförmige Mineralien, beispielsweise Wollastonit, Metall- und insbesondere Glasfasern verschiedener Länge, die gegebenenfalls geschlichtet sein können. Organische Füllstoffe, wie z.B. Kohle, Melamin, Kolophonium, Cyclopentadienylharze und Pfropfpolymerisate sowie Cellulosefasern, Polyamid-, Polyacrylnitril-, Polyurethan-, Polyesterfasern und organische Füllstoffe auf der Grundlage von aromatischen und/oder aliphatischen Dicarbonsäureestern und insbesondere Kohlenstoffasern.

Die anorganischen oder organischen Füllstoffe können einzeln oder als Gemische verwendet werden und werden der Reaktionsmischung vorteilhafterweise in Mengen von 0,5 bis 50 Gew.-%, vorzugsweise 1 bis 40 Gew.-%, bezogen auf das Gewicht der Komponenten (beispielsweise (b) bis (f)), einverleibt.

Die Erfindung soll nun anhand der nachstehenden, nicht limitierenden Beispiele erläutert werden.

### Beispiele:

### I. Phospholene

Allgemein wird frischdestilliertes Diacetyl oder Benzil tropfenweise unter Stickstoffatmosphäre bei 0-5°C mit einem Phosphit umgesetzt. Die Mischung wird mehrere Stunden bei 20°C gehalten und anschließend unter Vakuum destilliert.

Bei der Umsetzung von Diacetyl mit Trimethylphosphit entsteht 2,2,2-Trimethoxy-4,5-dimethyl-2,2-dihydro-1,3,2-dioxaphospholen, das einen Siedepunkt von 62°C bei 3 mbar und einen Brechungsindex von n²⁰_{D}= 1,4398 hat.

Bei der Umsetzung von Benzil mit Trimethylphosphit entsteht 2,2,2-Trimethoxy-4,5-diphenyl-2,2-dihydro-1,3,2-dioxaphospholen.

Bei der Umsetzung von Diacetyl mit Triphenylphosphit entsteht 2,2,2-Triphenoxy-4,5-dimethyl-2,2-dihydro-1,3,2-dioxaphospholen.

Bei der Umsetzung von Benzil mit Triphenylphosphit entsteht 2,2,2-Triphenoxy-4,5-diphenyl-2,2-dihydro-1,3,2-dioxaphospholen.

Bei der Umsetzung von Diacetyl mit Triethylphosphit entsteht 2,2,2-Triethoxy-4,5-dimethyl-2,2-dihydro-1,3,2-dioxaphospholen, das einen Siedepunkt von 58°C bei 2 mbar und einen Brechungsindex von n²²_{D}= 1,4383 hat.

### II. Hydantoingruppenhaltige Diisocyanate:

1. 431g (2,48 mol) 2,4-Toluyldiisocyanat (2,4-TDI) wurden in 200 ml trockenem Dichlormethan vorgelegt und bei Raumtemperatur unter Inertgas eine Lösung von 46g (0,219 mol) 2,2,2-Trimethoxy-4,5-dimethyl-2,2-dihydro-1,3,2-dioxaphospholen in 100 ml Dichlormethan zugetropft. Die Mischung wurde 24 Stunden unter Rückfluß gehalten und anschließend unter Normal-druck das Lösungsmittel abdestilliert. Im Vakuum wurde überschüssiges 2,4-TDI und das Reaktionsnebenprodukt Trimethylphosphat abdestilliert. Der Destillationsrückstand ist das Reaktionsprodukt 5-Acetyl-1,3-bis(3-isocyanato-4-methylphenyl)-5-methyl-hydantoin und stellt ein gelbes, zähes, allmählich erstarrendes Öl mit einem Schmelzpunkt > 320°C dar.
Der Isocyanatgehalt beträgt für das Reaktionsprodukt theoretisch 20,09% und für das Diisocyanat der Formel (I) mit n = 2 theoretisch 12,68%, gefunden wurden 20,3%.
Die Elementaranalyse für das Reaktionsprodukt ergab für C₂₂H₁₈N₄O₅(418,41) und für das Dimere n = 2 ergab für C₃₅H₃₀N₆O₈ (662,67):

| | C | H | N | O | P | Formel (I) |
|---|---|---|---|---|---|---|
| berechnet | 63,15 | 4,34 | 13,39 | 19,12 | 0,0 | n = 1 |
| berechnet | 63,44 | 4,56 | 12,68 | 19,32 | -/- | n = 2 |
| gefunden | 62,6 | 4,6 | 13,8 | 19,1 | 0,062 | gefunden |

Die Elementaranalyse für das Dimere ergab für C₂₄H₂₆N₄O₇ (482,50):

| | C | H | N | O |
|---|---|---|---|---|
| berechnet | 59,54 | 5,34 | 11,61 | 23,21 |
| gefunden | 58,3 | 5,3 | 12,8 | 24,2 |

Das ¹H- NMR Spektrum in CDCl₃ ergab folgende chemische Verschiebung δ in ppm:
1,78 (s, 3H, Me), 2,18, 2,22 (2s, 6H, Ar-Me), 2,31 (s, 3H, CO-Me), 7,1 (mc, 6H, aromat. H).
Das Massenspektrum ergab (EI): m/z = 173 (96%), 220 (41%), 375 (100%), 418 (33%, M⁺ Reaktionsprodukt), 662 (<1%, M⁺ Diisocyanat der Formel (I) mit n = 2.
2.1. 87,0g (0,5 mol) 2,4-TDI wurden in 300 ml trockenem Dichlormethan gelöst und mit 18,6g (0,0357 mol) 2,2,2-Triphenoxy-4,5-diphenyl-2,2-dihydro-1,3,2-dioxaphospholen umgesetzt. Die Mischung wurde 24 Stunden unter Rückfluß gehalten und anschließend unter Normaldruck das Lösungsmittel abdestilliert. Überschüssiges 2,4-TDI und das Reaktionsnebenprodukt Triphenylphosphat wurden bei 2mbar und 200°C abdestilliert. Der Destillationsrückstand ist das Reaktionsprodukt 5-Benzyl-1,3-bis(3-isocyanato-4-methyl-phenyl)-5-phenyl-hydantoin und ist ein gelbliches, langsam erstarrendes Öl mit einem Isocyanatgehalt von 17,3% (theoretisch 16,9%).
Die Elementaranalyse ergab für C₂₂H₁₈N₄O₅ (418,41):

| | C | H | N | O |
|---|---|---|---|---|
| berechnet | 74,7 | 4,67 | 11,8 | 10,1 |
| gefunden | 69,6 | 4,4 | 7,0 | 17,6 |

2.2. In analoger Weise wurden folgende Reaktionen durchgeführt:
909g 2,4-/2,6-Toluoldiisocyanat 80:20 wurden mit 95,0g 2,2,2-Trimethoxy-4,5-dimethyl-2,2-dihydro-1,3,2-dioxaphospholen zu dem entsprechenden Hydantoin-modifizierten Diisocyanat umgesetzt, das ein gelbes allmählich erstarrendes Öl mit einem Isocyanatgehalt von 21,3% (theoretisch 20,09%) ist.
Die Elementaranalyse ergab für C₂₂H₁₈N₄O₅ (418,41):

| | C | H | N | O | P |
|---|---|---|---|---|---|
| berechnet | 63,15 | 4,34 | 13,39 | 19,12 | 0,0 |
| gefunden | 62,3 | 4,7 | 13,8 | 18,5 | 0,048 |

2.3. 87g 2,4-/2,6-Toluoldiisocyanat 80:20 wurden mit 18,6g 2,2,2-Triphenoxy-4,5-diphenyl-2,2-dihydro-1,3,2-dioxaphospholen zu dem entsprechenden Hydantoin-modifizierten Diisocyanat, ein gelbes allmählich erstarrendes Öl mit einem Isocyanatgehalt von 15,3% (theoretisch 16,9%) umgesetzt.
2.4. 87g 2,4-/2,6-Toluoldiisocyanat 65:35 wurden mit 18,6g 2,2,2-Trimethoxy-4,5-dimethyl-2,2-dihydro-1,3,2-dioxaphospholen zu dem entsprechenden Hydantoin-modifizierten Diisocyanat umgesetzt. Man erhielt ein gelbliches sehr viskoses Öl mit einem Isocyanatgehalt von 19,0% (theoretisch 20,09%).
Die Elementaranalyse ergab für C₂₂H₁₈N₄O₅ (418,41):

| | C | H | N | O | P |
|---|---|---|---|---|---|
| berechnet | 63,15 | 4,34 | 13,39 | 19,12 | 0,0 |
| gefunden | 62,7 | 4,2 | 14,4 | 19,2 | 0,06 |

3. 42,0g (0,2 mol) Trimethoxy-4,5-dimethyl-2,2-dihydro-1,3,2-dioxaphospholen wurden bei Raumtemperatur unter Schutzgas zu einer Lösung von 250g (1,0 mol) 4,4'-Diphenylmethandiisocyanat (MDI) in 300 ml Dichlormethan getropft. Die Mischung wird 24 Stunden lang unter Rückfluß gehalten. Nach anschließender Abkühlung wurde das Lösungsmittel entfernt und überschüssiges MDI und entstandenes Trimethylphosphat bei 3-4 mbar und 180 bis 190°C im Dünnfilmverdampfer entfernt. Das Reaktionsprodukt wurde in einer Ausbeute von 91g (80%) als erstarrendes Öl mit einem Schmelzpunkt von 108°C erhalten. Ein Isocyanatgehalt von 11,5% (theoretisch 14,7%) wurde ermittelt.
Auch von diesem Produkt wurde das Bisurethan in der unter...angegebenen Weise synthetisiert, das Bisurethan besitzt einen Schmelzpunkt von 101° - 121° C.
Die Elementaranalyse ergab:

| | C | H | O | N |
|---|---|---|---|---|
| berechnet | 71,6 | 4,6 | 14,0 | 9,8 |
| gefunden | 66,6 | 5,8 | 18,1 | 8,7 |

4. Diisocyanat 1:
50,5 g (0,2 mol) 2,2,2-Triethoxy-4,5-dimethyl-2,2-dihydro-1,3,2-dioxaphospholen wurden bei Raumtemperatur unter Schutzgas zu einer Lösung von 200 g (0,80 mol) polymerem 4,4'-Diphenylmethandiisocyanat (Polymer-MDI Viskosität 200 mPa·s/25°C) in 300 ml Dichlormethan getropft. Die Mischung wurde 24 Stunden unter Rückfluß gehalten. Anschließend wurde die Mischung abgekühlt und unter Normaldruck das Lösungsmittel abdestilliert. Ohne weitere Destillation wurde ein Öl mit einem Isocyanatgehalt von 14,8 % und einer Viskosität von 29 Pa·s (23°C) erhalten. Das Öl enthielt außerdem das Reaktionsnebenprodukt Triethylphosphat.
Nach Abdestillieren des Reaktionsnebenproduktes Triethylphosphat im Dünnschichtverdampfer und unter Vakuum, hatte das Produkt einen Isocyanatgehalt von 18,9 % und eine Viskosität von 77 Pa·s (60°C).
5. Diisocyanat 2:
50,5 g (0,2 mol) 2,2,2-Triethoxy-4,5-dimethyl-2,2-dihydro-1,3,2-dioxaphospholen wurden bei Raumtemperatur unter Schutzgas zu einer Lösung von 300,4 g (1,20 mol) Polymer-MDI (Cupranat M-20S) in 300 ml Dichlormethan getropft. Die Reaktion wurde analog zu der für Diisocyanat 1 durchgeführt. Ein Öl mit einem Isocyanatgehalt von 18,6% (19,1%) und einer Viskosität von 6 Pa·s bei 23°C (0,170 Pa·s bei 60°C) wurde erhalten. Nach Abdestillieren des Reaktionsnebenproduktes Triethylphosphat im Dünnschicht-verdampfer und unter Vakuum, hatte das Produkt einen Isocyanatgehalt von 21,6% (22,5%) und eine Viskosität von 3,87 Pa·s bei 60°C (5,51 Pa·s bei 60°C).
6. Diisocyanat 3:
50,5 g (0,2 mol) 2,2,2-Triethoxy-4,5-dimethyl-2,2-dihydro-1,3,2-dioxaphospholen wurden bei Raumtemperatur unter Schutzgas zu einer Lösung von 250 g (1,00 mol) 4,4'-Diphenylmethandiisocyanat (MDI) in 300 ml Dichlormethan getropft. Die Reaktion wurde analog zu der für Diisocyanat 1 durchgeführt. Ein Öl mit einem Isocyanatgehalt von 18,0% und einer Viskosität von 0,091 Pa·s (23°C) erhalten. Nach teilweisem Abdestillieren des Reaktionsnebenproduktes Triethylphosphat und überschüssigem 2,2,2-Triethoxy-4,5-dimethyl-2,2-dihydro-1,3,2-dioxaphospholen im Dünnschichtverdampfer und unter Vakuum, hatte das Produkt einen Isocyanatgehalt von 20,8% und eine Viskosität von 0,69 Pa·s (23°C).
7. Diisocyanat 4:
50,5 g (0,2 mol) 2,2,2-Triethoxy-4,5-dimethyl-2,2-dihydro-1,3,2-dioxaphospholen wurden bei Raumtemperatur unter Schutzgas zu einer Lösung von 200 g (0,80 mol) MDI in 300 ml Dichlormethan getropft. Die Reaktion wurde analog zu der für Diisocyanat 1 durchgeführt. Ein Öl mit einem Isocyanatgehalt von 17,5% und einer Viskosität von 0,095 Pa·s bei 23°C (0,170 Pa·s bei 60°C) erhalten. Nach Abdestillieren des Reaktionsnebenproduktes Triethylphosphat im Dünnschichtverdampfer und unter Vakuum, hatte das Produkt einen Isocyanatgehalt von 21,1% und eine Viskosität von 0,7 Pa·s (23°C).
8. Standard-Diisocyanat :
Ein Gemisch aus 4,4'-Diphenylmethandiisocyanat (MDI) und polymerem 4,4'-Diphenylmethandiisocyanat (Polymer-MDI) mit einem Isocyanatgehalt von 31,7% und einer Viskosität von 0,209 Pa·s (25°C).
Diisocyanat Mischung **1**: 80:20 Mischung aus dem Standard-Diisocyanat und Diisocyanat 1
Diisocyanat Mischung **2**: 80:20 Mischung aus dem Standard-Diisocyanat und Diisocyanat 2
Diisocyanat Mischung **3**: 80:20 Mischung aus dem Standard-Diisocyanat und Diisocyanat 3
Diisocyanat Mischung **4**: 80:20 Mischung aus dem Standard-Diisocyanat und Diisocyanat 4

Zur Herstellung der hier beschriebenen Mischungen wird ein Reaktormischbehälter mit einem Flügelrührer eingesetzt.

### III. Polyole:

Die im Folgenden angegebenen Mengenangaben (Teile) beziehen sich auf Gewichtsteile.

Polyol 1 (Lupranol® L 3422):
25,2 Teile Sorbit, 74,8 Teile Propylenoxid, in Gegenwart von KOH als Katalysator und 0,5 Teile Wasser. Die Hydroxylzahl beträgt 495 mg KOH/g, die Viskosität 17,9 Pa·s (20°C) und die Funktionalität 5.

Polyol 2 (Lupranol® VP 9196):
1 Teil Saccharose, 1 Teil Pentaerythrit, 2 Teile Diethylenglykol, Propylenoxid, 0,5 Teile Wasser als und KOH als Katalysator. Die Hydroxylzahl beträgt 400 mg KOH/g, die Viskosität 2,2 Pa·s (20°C).

Polyol 3 (Lupraphen® 8004):
Ein Polyesteralkohol aus Adipinsäure, Phthalsäureanhydrid und Ölsäure im Verhältnis 1:2:1 und 1,1,1-Trimethylolpropan mit einem Zahlenmittel des Molekulargewichts (Mₙ) von 530 g/Mol. Die Hydroxylzahl beträgt 385 mg KOH/g, die Viskosität 1,37 Pa·s (70°C).

Polyol 4 (Lupranol® 2045):
Glycerol als, Propylenoxid als erster Block und Ethylenoxid als Endblock. Die Hydroxylzahl beträgt 35 mg KOH/g, die Viskosität 0,85 Pa·s (20°C). Das Masseverhältnis von Ethylenoxid zu Propylenoxid beträgt 6,4.

### IV. Polyurethan-Hartschaum:

Zur Prüfung der Polyurethan-Hartschaumstoffe wurde ein Formkörper hergestellt.
672 g eines Gemisches einer Komponente A bestehend aus
53,56 Teile Polyol 1
28,04 Teile Polyol 2
5,61 Teile Dipropylenglykol
1,87 Teile Glycerin
1,4 Teile Polyethersilaxane der TH Goldschmidt als Stabilisatorgemisch
2,14 Teile eines Katalysatorgemisches aus tertiären di-Methylcyclohexylamin
0,84 Teile Wasser
6,54 Teile Cyclopentan

und einer Komponente B, die dem zu untersuchenden Diisocyanat entspricht, wurden in ein auf 45°C temperiertes Gehäuse (Formwerkzeug; 300*400*80mm) gegossen, das anschließend fest verschlossen wurde. Die Gesamtdichte des Formkörpers betrug 70 ± 1 kg/m³. Die Entformungszeit betrug 30 Minuten. Nach 24 Stunden wurden zur Messung der Wärmeleitfähigkeit und der Wärmeformbeständigkeit Prüfkörper aus dem Innern des Schaumkörpers herausgesägt.

Die Wärmeformbeständigkeit wurde in Anlehnung an die DIN-Norm 18164 an Prüfkörpern der Abmessung 50x50x50mm nach einer Belastung von 0,04 N/mm² für die Dauer von 24 Stunden als Verformung in Prozent bei 170°C gemessen.

Bei den einzelnen Rezepturen für die Polyurethan-Hartschaumstoffe wurde das molare Verhältnis zwischen Isocyanatgruppe und wasserstoffaktiven Gruppen konstant auf 118 gehalten und die Menge des Isocyanats für die Komponente B variiert:
Polyurethan **1**: 100 Teile A + 155 Teile Diisocyanat-Mischung 1
Polyurethan **2**: 100 Teile A + 152 Teile Diisocyanat -Mischung 2
Polyurethan **3**: 100 Teile A + 157 Teile Diisocyanat -Mischung 3
Polyurethan **4**: 100 Teile A + 153 Teile Diisocyanat -Mischung 4

Daraus ergab sich für die Prüfkörper aus dem Formkörper mit den in Tabelle 1 aufgeführten Eigenschaften:

**Tabelle 1:**

| Polyurethan | **1** | **2** | **3** | **4** |
|---|---|---|---|---|
| Topfzeit [s] | 12 | 12 | 13 | 12 |
| Abbindezeit [s] | 64 | 64 | 63 | 61 |
| Steigzeit [s] | 102 | 100 | 99 | 94 |
| Rohdichte freigeschäumt [kg/m³] | 51,7 | 55,7 | 51,1 | 56,9 |
| Gesamtdichte [kg/m³] | 71 | 71 | 71 | 71 |
| Druckfestigkeit [N/mm²] | 0,59 | 0,59 | 0,49 | 0,49 |
| Wärmeleitfähigkeit nach 7d [mW/mK] | 24,9 | 24,7 | 24,6 | 23,6 |
| Wärmeformbeständigkeit bei 170°C in % | **11** | **12,2** | | **16,8** |

Polyurethan/Polyisocyanurat-Hartschaumstoffe durch Becherverschäumung wurden hergestellt aus einer Komponente A:
25,6 Teile Polyol 3
31,65 Teile Polyol 4
16,6 Teile Dipropylenglykol
2,58 Teile Polyethersiloxane der TH Goldschmidt als Stabilisatorgemisch

2,71 Teile Ethylenglykol
0,38 Teile Wasser
2,41 Teile Kaliumacetat
0,27 Teile Katalysatorgemisch aus tertiären Aminen
17,8 Teile Cyclopentan
und einer Komponente B, die dem zu untersuchenden Diisocyanat entspricht und die zusammen auf 20 ± 0,5°C temperiert wurden. 78g der Mischung wurden in einem Pappbecher mit einem Volumen von ca. 660 ml für 10 Sekunden bei 1750 Umdrehungen/min verrührt. Dabei entsteht ein Schaum. Am aufsteigenden Schaum werden Start-, Steig- und Abbindezeit und vom ausgehärteten Schaum die Rohdichte in der dem Fachmann bekannten Weise gemessen.

Bei den einzelnen Rezepturen für die Polyurethan/Polyisocyanurat-Hartschaumstoffe wurde das molare Verhältnis zwischen Isocyanatgruppe und Hydroxidgruppe konstant auf 418 gehalten und die Menge des Isocyanats für die Komponente B variiert.
Polyurethan **5**: 100 Teile A + 347 Teile Diisocyanat-Mischungen 1
Polyurethan **6**: 100 Teile A + 340 Teile Diisocyanat-Mischungen 2
Polyurethan **7**: 100 Teile A + 351 Teile Diisocyanat-Mischunge 3
Polyurethan **8**: 100 Teile A + 343 Teile Diisocyanat-Mischunge 4

Für die Prüfkörper aus der Becherverschäumung ergaben sich die Eigenschaften gem. Tabelle 2:

**Tabelle 2:**

| Polyurethan | 5 | 6 | 7 | 8 |
|---|---|---|---|---|
| Topfzeit[s] | 15 | 15 | 16 | 17 |
| Abbindezeit [s] | 30 | 29 | 33 | 33 |
| Steigzeit [s] | 51 | 45 | 51 | 50 |
| Rohdichte freigeschäumt [kg/m³] | 51,9 | 56,6 | 51,6 | 53,5 |
| Wärmeformbeständigkeit bei 200°C in % | 10,1 | 6,8 | 25,0 | 17,2 |

### V. Polyurethanlacke

Hydantoinhaltiges 1,6-Hexamethylendüsocyanat:
168 g 1,6-Hexamethylendiisocyanat (HDI) wurden in 350 ml Dichlormethan unter Schutzgas vorgelegt. Bei Raumtemperatur wurden langsam 18,6 g in 50 ml Dichlormethan gelösten 2,2,2-Trimethoxy-4,5-dimethyl-2,2-dihydro-1,3,2-dioxa-phospholen zugesetzt. Anschließend wurde das Gemisch 24 Stunden unter Rückfluß gehalten. Nachdem das Lösungsmittel unter Normaldruck abdestilliert wurde, wurden bei 5 mbar und 150°C überschüssiges HDI und entstandenes Trimethylphosphat abdestilliert. Der Destillationsrückstand (Reaktionsprodukt), 5-Acetyl-1,3-bis(6-isocyanato-hexamethylen)-5-methyl-hydantoin, ist eine blaßgelbe Flüssigkeit mit einer Viskosität von 0,450 Pa·s (23°C). Der Isocyanatgehalt für das Hydantoin-modifizierte 1,6-Hexamethylendi-isocyanat betrug theoretisch 20,7% und experimentell ermittelt 17,5%.

Das Hydantoin-modifizierte 1,6-Hexamethylendiisocyanat wurde in pigmentierten Polyurethan-Lacken (Pigment TiO₂ Kronos® 2310 der Fa. Kronos Titan GmbH, Leverkusen) auf der Basis eines hydroxylgruppenhaltigen Acrylatharzes mit einem Gewichtsmittel des Molekulargewichts von ca. 6000 und einer OH-Zahl von 135 (mg KOH/g Harz; Lumitol® H 136 der Fa. BASF) und einem Acrylatharz mit einer mittlerem Molmasse von ca. 8000 und einer OH-Zahl von 85 (Lumitol® H 85 der Fa. BASF) eingesetzt.

Für den Vergleich wurden die Lacke mit einem niederviskosen aliphatischen Polyisocyanat auf HDI-Basis (Isocyanurat-Typ; Basonat® PLR 8900 der Fa. BASF) mit vergleichbarem Isocyanatgehalt und einer Viskosität von 1,195 Pa·s (23°C) vernetzt.

Die untersuchten Lacke hatten folgende Zusammensetzung:
**Lack 1**: 100 T Lumitol® H 85, 56,36 T TiO₂ Kronos® 2310, 25,45 T n-Butylacetat/Xylol 2:3 und 15,45 T Hydantoin-modifiziertes 1,6-Hexamethylendiisocyanat
**Lack 2**: 100 T Lumitol® H 85, 56,36 T TiO₂ Kronos® 2310, 25,79 T n-Butylacetat/Xylol 2:3 und 15,79 T Basonat® PLR 8900
**Lack 3**: 100 T Lumitol® H 136, 80,98 T TiO₂ Kronos® 2310, 71,22 T n-Butylacetat/Xylol 2:3 und 31,22 T Hydantoin-modifiziertes 1,6-Hexamethylendiisocyanat
**Lack 4**: 100 T Lumitol® H 136, 80,98 T TiO₂ Kronos® 2310, 71,93 T n-Butylacetat/Xylol 2:3 und 31,93 T Basonat® PLR 8900

Die Lacke, bei denen hydantoingruppenhaltige 1,6-Hexamethylendiisocyanat eingesetzt wurden zeigen im Vergleich zu dem handelsüblichen Polyisocyanat bessere Elastizität .

**Tabelle 3:**

| Lack auf | 1 | 2 | 3 | 4 |
|---|---|---|---|---|
| Erichsentiefung [mm] DIN 53156 | 8,9/9,2 | 7,1/7,5 | 7,3/7,9 | 7,0/7,1 |

Die Lacke 1 - 4 wurden auf Eisen-Bonder des Typs 26/60-OC der Firma Chemetall GmbH Frankfurt aufgerakelt. Die Naßfilmdicke betrug 150 µm.

## Patentansprüche

1. Diisocyanat der Formel (I) 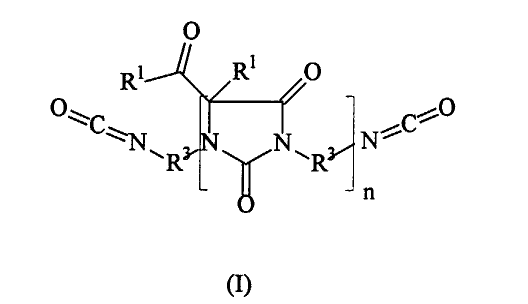 wobei R¹ ein C₁- bis C₁₀-Kohlenwasserstoffrest und R³ eine C₁- bis C₁₂-Kohlenwasserstoffgruppe bedeutet und n einer ganzen Zahl im Bereich von 1 bis 10 entspricht.

2. Diisocyanat der Formel (I) nach Anspruch 1, wobei R³ eine Alkylengruppe mit 3 bis 12 Kohlenstoffatomen, eine Cycloalkenylgruppe mit 3 bis 12 Kohlenstoffatomen oder eine Arylengruppe mit 7 bis 12 Kohlenstoffatomen bedeutet.

3. Verfahren zur Herstellung von Diisocyanat, wobei Phospholene der Formel (II), in der R² ein C₁- bis C₁₀-Kohlenwasserstoffrest bedeutet, mit Diisocyanaten der Formel (III) umgesetzt werden.

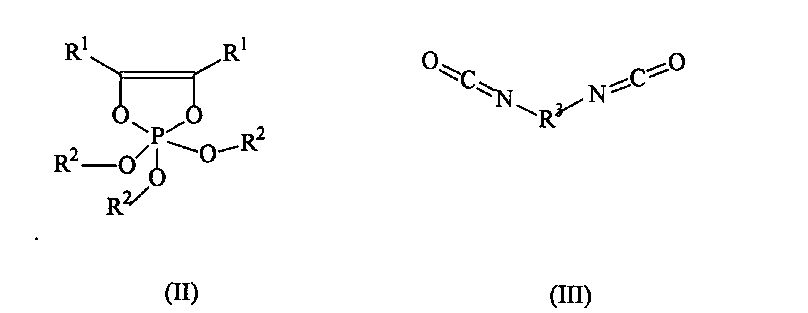

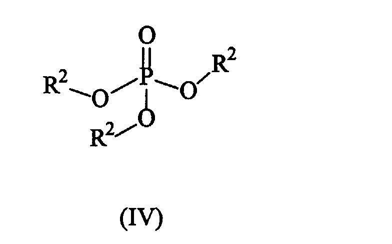

4. Verfahren gemäß Anspruch 3, wobei das bei der Reaktion entstehende Phosphat der Formel (IV) vollständig aus dem Reaktionsgemisch entfernt wird.

5. Zusammensetzung, erhältlich nach einem Verfahren gemäß Anspruch 3 oder 4 mindestens, enthaltend Diisocyanat der Formel (I) nach Anspruch 1, Diisocyanat der Formel (III) .

6. Polyurethan, erhältlich aus mindestens den Bestandteilen
a) mindestens als organisches Polyisocyanat ein Diisocyanat der Formel (I) nach Anspruch 1 oder mindestens einer Zusammensetzung nach Anspruch 5 oder deren Mischung.
b) mindestens als organisches Polyol ein Diol als organische Verbindung mit mindestens zwei reaktiven Wasserstoffatomen.

7. Polyurethan, erhältlich aus mindestens den Bestandteilen
a) mindestens als organisches Polyisocyanat ein Diisocyanat der Formel (I) nach Anspruch 1 oder Zusammensetzung nach Anspruch 5 oder deren Mischung.
b) Acrylatharz mit mindestens zwei Hydroxylgruppen pro Molekül und einem Molekulargewicht von 300 bis 20000 g/mol.

8. Polyurethan nach Anspruch 7, zusätzlich Pigmente enthaltend.

9. Verwendung eines Polyurethans nach einem der Ansprüche 6 bis 8 für die Herstellung von Fasern, Folien, Schäumen, Formmassen, Beschichtungen und Lacke.

10. Fasern, Folien, Schäume, Formmassen, Beschichtungen und Lacke, enthaltend Polyurethan nach einem der Ansprüche 6 bis 8.
